# FASCICULE DE BREVET EUROPEEN

(11) **EP 1 127 876 B1**
(45) Date de publication et mention de la délivrance du brevet: **13.11.2002**
(21) Numéro de dépôt: 01400484.0
(22) Date de dépôt: 23.02.2001
(51) Int. Cl.: C07D 209/44

(54) **Procédé de préparation de l'isoindoline**
Verfahren zur Herstellung von Isoindolin
Process for the preparation of isoindoline

(30) Priorité: 25.02.2000 FR 0002383
(43) Date de publication de la demande: 29.08.2001
(73) Titulaire: LES LABORATOIRES SERVIER, 92200 Neuilly sur Seine (FR)
(72) Inventeur: Souvie, Jean-Claude, 76600 Le Havre (FR); Fugier, Claude, 76210 Gruchet le Valasse (FR); Lecouve, Jean-Pierre, 76600 Le Havre (FR)

(56) Documents cités:
- L. KH. FREIDLIN ET AL: "Formation of nitrogen-containing heterocycles in the catalytic hydrogenation of succinonitrile and phthalonitrile" IZVESTIA ENG. ED., - 1959 pages 1778-1780, XP000974459

## Description

La présente invention concerne un nouveau procédé de préparation de l'isoindoline.

L'isoindoline est un intermédiaire de synthèse largement utilisé, notamment dans la préparation de principes actifs pharmaceutiques.

En particulier, l'isoindoline est un intermédiaire important dans la synthèse de l'acide 2-(S)-benzyl-4-oxo-4-(cis-perhydroisoindol-2-yl)-butyrique de formule (I) : 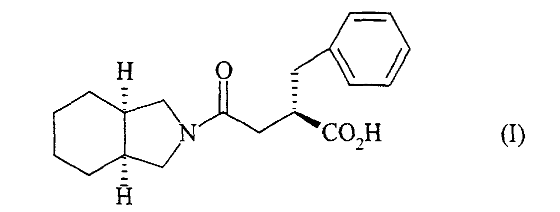 de ses sels pharmaceutiquement acceptables et de ses hydrates.

Le composé de formule (I), ainsi que ses sels d'addition et ses hydrates, possèdent des propriétés pharmacologiques particulièrement intéressantes. Ce sont des insulinosécréteurs très puissants, ce qui les rend utiles dans le traitement des diabètes non insulino-dépendants. Le composé de formule (I), sa préparation et son utilisation en thérapeutique ont été décrits dans le brevet EP 0 507 534. Sa préparation industrielle est décrite dans le brevet WO 99/01430. Compte-tenu de l'intérêt pharmaceutique de ce composé, il était important de pouvoir accéder à l'isoindoline intermédiaire avec un procédé de synthèse industrielle performant.

Plusieurs méthodes de préparation de l'isoindoline sont déjà connues.
Toutefois, aucun procédé décrit dans la littérature ne permet l'obtention de l'isoindoline avec une pureté et un rendement satisfaisants, tout en étant facilement transposable à l'échelle industrielle et avantageux du point de vue économique.

La préparation de l'isoindoline par réduction électrolytique ou chimique du phtalimide est décrite dans les journaux Bull. Soc. Chim. France 1956, 906-910, J. Pharm. Sci. 1964, 53 (8), 981 et J. Org. Chem. 1988, 53 (22), 5381-5383.

Toutefois, ces procédés ne permettent pas d'obtenir l'isoindoline avec un rendement supérieur à 50 %.

La préparation de l'isoindoline par cyclisation de l'α,α'-dibromo-xylène en présence de p-toluènesulfonylamine, suivie de la déprotection de la *N*-(p-toluènesulfonyl)-isoindoline obtenue est décrite dans les journaux J. Org. Chem. 1957, 22, 1255-6 et Org. Synth. Collect. Vol. V, 406-408 et 1064-1066.
Cette méthode, outre son faible rendement (moins de 50 %), présente l'inconvénient d'utiliser une matière première extrêmement lacrymogène.

Les brevets FR 1 577 845 et FR 1 578 582 décrivent la préparation de l'isoindoline par réaction de l'α,α'-dichlorobenzène avec l'hexaméthylènetétramine, suivie du traitement en milieu HCl ou SO₂ du sel d'ammonium obtenu, puis de la cyclisation en milieu basique du dérivé d'o-chlorométhyl-benzylamine obtenu.
Cette méthode est particulièrement longue et ne permet pas d'obtenir l'isoindoline avec un rendement satisfaisant.

Le journal Izvestia Eng. Ed. 1959, 1778-80, décrit la synthèse de l'isoindoline par hydrogénation du phtalonitrile à 100-120 atm dans un mélange dioxane/ammoniac, en présence de nickel ou de cobalt et à 100°C.

Ce procédé présente plusieurs inconvénients. Les rendements annoncés (91 à 98 %) n'ont pu être reproduits. Après élimination du catalyseur par filtration et élimination du solvant par distillation, aucun composé n'a pu être distillé du milieu chauffé à 120°C sous vide de 20 mbars. Par ailleurs, la présence d'ammoniac dans le milieu réactionnel nécessite lors du traitement à l'échelle industrielle une installation particulière pour protéger l'environnement.

Compte-tenu de l'intérêt de l'isoindoline en tant qu'intermédiaire de synthèse de principes actifs pharmaceutiques et notamment de l'acide 2-(S)-benzyl-4-oxo-4-(cis-perhydroisoindol-2-yl)-butyrique, et de l'absence d'un procédé permettant son obtention avec un bon rendement et une pureté satisfaisante, partant de matières premières peu onéreuses mais évitant l'utilisation d'ammoniac, la demanderesse a entrepris des recherches approfondies qui ont abouti à la mise au point d'un nouveau procédé de préparation de l'isoindoline.

Ce procédé permet l'obtention de l'isoindoline en une seule étape, par simple hydrogénation catalytique du phtalonitrile, produit commercial, sans addition d'ammoniac, avec un rendement supérieur à 75 % et avec une très bonne pureté.

Pour obtenir ce résultat, les conditions opératoires suivantes doivent être appliquées :
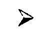 Le catalyseur utilisé est le Pt/C à 5 %. En effet, il est apparu de manière surprenante que parmi les catalyseurs généralement employés, seul le Pt/C permettait d'obtenir l'isoindoline dans une durée de réaction raisonnable (tableau 1).
La quantité de Pt/C utilisée représente 10 à 25 %, préférentiellement 20 % du poids du phtalonitrile.

**Tableau 1 :**

| *Catalyseur* | *Temps d'hydrogénation* | *% isoindoline* |
|---|---|---|
| Pd/C | 20 h | 1 |
| Ni Raney | 20 h | 0 |
| Rh/C | 20 h | 0 |
| Ru/C | 20 h | 0 |
| Pt/C | 6 h | 89,9 |

*Conditions de réaction : tétrahydrofurane, 20 % en poids de catalyseur, 60°C, 180 bars d'hydrogène*
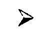 Le solvant utilisé est le tétrahydrofurane, un mélange tétrahydrofurane /eau dont la teneur en eau est inférieure à 10 %, préférentiellement inférieure à 5 %, ou le diméthoxyéthane. En effet, il est apparu de façon surprenante que seuls le tétrahydrofurane (utilisé seul ou en présence d'une quantité limitée d'eau) et le diméthoxyéthane permettaient d'obtenir un taux de conversion satisfaisant (tableau 2).

**Tableau 2:**

| *Solvant* | *Temps d'hydrogénation* | *% isoindoline* |
|---|---|---|
| tétrahydrofurane | 6 h | 89,9 |
| tétrahydrofurane/eau 98/2 | 6 h | 89,3 |
| diméthoxyéthane | 9 h | 86,2 |
| dioxane | 20 h | 40 |
| éthanol | 20 h | 49 |
| diméthylformamide | 20 h | 0 |

*Conditions de réaction : catalyseur Pt*/*C (20 % en poids), 60°C, 180 bars d'hydrogène*
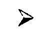 La pression d'hydrogène à l'intérieur du réacteur est comprise entre 100 et 180 bars et préférentiellement entre 150 et 180 bars.
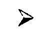 La température du milieu réactionnel est comprise entre 30 et 100°C et préférentiellement entre 50 et 70°C.

L'isoindoline obtenue dans ces conditions peut être ensuite facilement isolée de son milieu réactionnel par distillation, puis purifiée par précipitation sous forme de chlorhydrate dans un solvant tel que, par exemple, l'éthanol ou l'acétate d'éthyle.

Le chlorhydrate d'isoindoline ainsi obtenu a une très bonne pureté et contient notamment moins de 1,5 %, préférentiellement moins de 0,2 % de 2-méthylbenzylamine, ce qui rend son emploi particulièrement avantageux dans la synthèse de principes actifs tels que le composé de formule (I).

A titre d'illustration, la réduction énantiosélective par hydrogénation catalytique de l'isoindoline obtenue selon le procédé de l'invention permet de conduire au cis-perhydroisoindole avec une pureté et un rendement très satisfaisants. Celui-ci, mis en réaction avec l'anhydride de formule (II) : 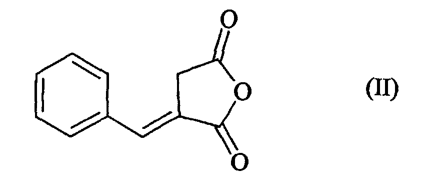 conduit au composé de formule (III) : 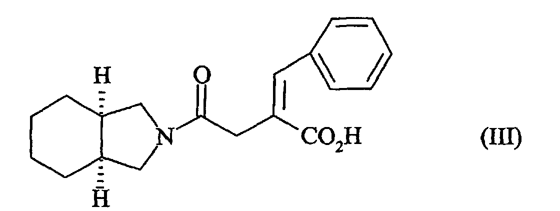 dont l'hydrogénation catalytique en présence d'un catalyseur asymétrique conduit au composé de formule (I).

Les exemples suivants illustrent l'invention.

La pureté des composés a été déterminée par chromatographie en phase gazeuse sur colonne OPTIMA-5 amine® (Macherey-Nagel), avec une détection FID (à ionisation de flamme) à 280°C.

### EXEMPLE 1 : Isoindoline

La réaction est effectuée dans un autoclave. A 100 g de phtalonitrile en solution dans le tétrahydrofurane sont ajoutés 20 g de platine à 5 % sur charbon. Après une purge à l'azote, le milieu est chauffé à 60°C et une pression d'hydrogène de 180 bars est appliquée pendant 5 à 6 heures. Après décompression et purge à l'azote, le catalyseur est éliminé par filtration. Le tétrahydrofurane est distillé du filtrat à pression atmosphérique, puis l'isoindoline est distillée à son tour du résidu sous un vide de 23 mbars, à une température de 100°C.

L'isoindoline est ainsi obtenue avec un rendement de 75 % et une pureté de 89 %.

### EXEMPLE 2 : Chlorhydrate de l'isoindoline

A 69 g d'isoindoline obtenue dans l'Exemple 1 en solution dans 458 ml d'acétate d'éthyle est ajoutée une solution d'acide chlorhydrique 2,5 N dans l'acétate d'éthyle. Le solide obtenu est récupéré par filtration, lavé par de l'acétate d'éthyle puis séché en étuve. Le chlorhydrate d'isoindoline est ainsi obtenu avec un rendement de 82 % et une pureté de 98,5 % avec moins de 1,5 % de 2-méthylbenzylamine.

## Revendications

1. Procédé de synthèse de l'isoindoline **caractérisé en ce que** l'on soumet une solution de phtalonitrile dans le tétrahydrofurane, dans un mélange tétrahydrofurane/eau, ou dans le diméthoxyéthane à une pression d'hydrogène comprise entre 100 et 180 bars, à une température comprise entre 30 et 100°C, et en présence de Pt/C à 5 %.

2. Procédé selon la revendication 1 **caractérisé en ce que** le solvant est le tétrahydrofurane.

3. Procédé selon la revendication 1 **caractérisé en ce que** le solvant est un mélange tétrahydrofurane/eau dont la teneur en eau n'excède pas 10 %.

4. Procédé selon la revendication 3 **caractérisé en ce que** le solvant est un mélange tétrahydrofurane/eau dont la teneur en eau n'excède pas 5 %.

5. Procédé selon la revendication 1 **caractérisé en ce que** le solvant est le diméthoxyéthane.

6. Procédé selon l'une quelconque des revendications 1 à 5 **caractérisé en ce que** la pression d'hydrogène est comprise entre 150 et 180 bars.

7. Procédé selon l'une quelconque des revendications 1 à 6 **caractérisé en ce que** la température est comprise entre 50 et 70°C.

8. Procédé selon l'une quelconque des revendications 1 à 7 **caractérisé en ce que** la quantité de Pt/C utilisée représente entre 10 et 25 % du poids de phtalonitrile engagé.

9. Procédé selon la revendication 8 **caractérisé en ce que** la quantité de Pt/C utilisée représente 20 % du poids de phtalonitrile engagé.

10. Procédé selon l'une quelconque des revendications 1 à 9 **caractérisé en ce que** le taux de 2-méthylbenzylamine dans l'isoindoline obtenue ne dépasse pas 1,5 %.

11. Procédé selon la revendication 10 **caractérisé en ce que** le taux de 2-méthylbenzylamine dans l'isoindoline obtenue ne dépasse pas 0,2 %.

12. Procédé de préparation de l'acide 2-(S)-benzyl-4-oxo-4-(cis-perhydroisoindol-2-yl)-butyrique par hydrogénation catalytique énantiosélective de l'isoindoline, suivie de la réaction du composé obtenu avec l'anhydride de formule (II) : 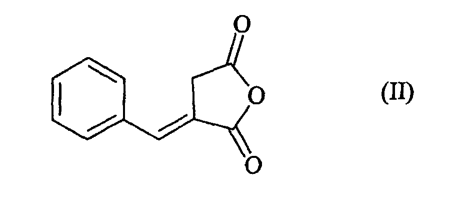 suivie de l'hydrogénation catalytique en présence d'un catalyseur asymétrique du composé (III) ainsi obtenu : 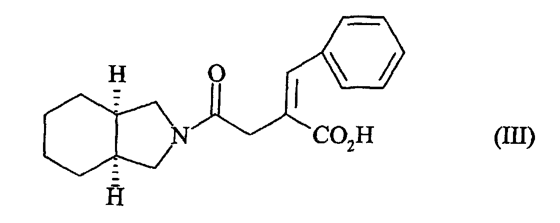 **caractérisé en ce que** l'isoindoline de départ est préparée selon le procédé de la revendication 1.

## Claims

1. Process for the synthesis of isoindoline, **characterised in that** a solution of phthalonitrile in tetrahydrofuran, in a mixture of tetrahydrofuran/water, or in dimethoxyethane is subjected to a hydrogen pressure of from 100 to 180 bars, at a temperature of from 30 to 100°C, and in the presence of 5% Pt/C.

2. Process according to claim 1, **characterised in that** the solvent is tetrahydrofuran.

3. Process according to claim 1, **characterised in that** the solvent is a mixture of tetrahydrofuran/water in which the water content does not exceed 10 %.

4. Process according to claim 3, **characterised in that** the solvent is a mixture of tetrahydrofuran/water in which the water content does not exceed 5 %.

5. Process according to claim 1, **characterised in that** the solvent is dimethoxyethane.

6. Process according to any one of claims 1 to 5, **characterised in that** the hydrogen pressure is from 150 to 180 bars.

7. Process according to any one of claims 1 to 6, **characterised in that** the temperature is from 50 to 70°C.

8. Process according to any one of claims 1 to 7, **characterised in that** the amount of Pt/C used is from 10 to 25 % of the weight of phthalonitrile employed.

9. Process according to claim 8, **characterised in that** the amount of Pt/C used is 20 % of the weight of phthalonitrile employed.

10. Process according to any one of claims 1 to 9, **characterised in that** the amount of 2-methylbenzylamine in the isoindoline obtained does not exceed 1.5 %.

11. Process according to claim 10, **characterised in that** the amount of 2-methylbenzylamine in the isoindoline obtained does not exceed 0.2 %.

12. Process for the preparation of 2-(S)-benzyl-4-oxo-4-(cis-perhydroisoindol-2-yl)-butyric acid by enantioselective catalytic hydrogenation of isoindoline, followed by reaction of the resulting compound with the anhydride of formula (II): 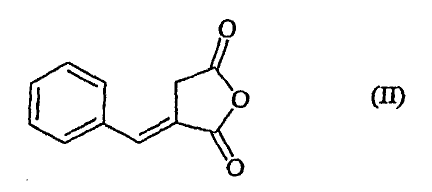 followed by catalytic hydrogenation, in the presence of an asymmetric catalyst, of compound (III) so obtained: 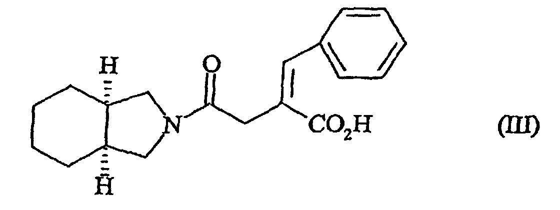 **characterised in that** the starting isoindoline is prepared according to the process of claim 1.

## Patentansprüche

1. Verfahren zur Synthese von Isoindolin, **dadurch gekennzeichnet, daß** man eine Lösung von Phthalonitril in Tetrahydrofuran, in einer Tetrahydrofuran/Wasser-Mischung oder in Dimethoxyethan in Gegenwart von 5 % Pt/C bei einer Temperatur zwischen 30 und 100°C einem Wasserstoffdruck zwischen 100 und 180 bar aussetzt.

2. Verfahren nach Anspruch 1, **dadurch gekennzeichnet, daß** das Lösungsmittel Tetrahydrofuran ist.

3. Verfahren nach Anspruch 1, **dadurch gekennzeichnet, daß** das Lösungsmittel eine Tetrahydrofuran/Wasser-Mischung ist, dessen Wassergehalt 10 % nicht übersteigt.

4. Verfahren nach Anspruch 3, **dadurch gekennzeichnet, daß** das Lösungsmittel eine Tetrahydrofuran/Wasser-Mischung ist, dessen Wassergehalt 5 % nicht übersteigt.

5. Verfahren nach Anspruch 1, **dadurch gekennzeichnet, daß** das Lösungsmittel Dimethoxyethan ist.

6. Verfahren nach einem der Ansprüche 1 bis 5, **dadurch gekennzeichnet, daß** der Wasserstoffdruck zwischen 150 und 180 bar liegt.

7. Verfahren nach einem der Ansprüche 1 bis 6, **dadurch gekennzeichnet, daß** die Temperatur zwischen 50 und 70°C liegt.

8. Verfahren nach einem der Ansprüche 1 bis 7, **dadurch gekennzeichnet, daß** die verwendete Pt/C-Menge 10 bis 25 % des Gewichts des eingesetzten Phthalonitrils beträgt.

9. Verfahren nach Anspruch 8, **dadurch gekennzeichnet, daß** die verwendete Pt/C-Menge 20 % des Gewichts des eingesetzten Phthalonitrils beträgt.

10. Verfahren nach einem der Ansprüche 1 bis 9, **dadurch gekennzeichnet, daß** der 2-Methylbenzylamin-Gehalt in dem erhaltenen Isoindolin 1,5 % nicht übersteigt.

11. Verfahren nach Anspruch 10, **dadurch gekennzeichnet, daß** der 2-Methylbenzylamin-Gehalt in dem erhaltenen Isoindolin 0,2 % nicht übersteigt.

12. Verfahren zur Herstellung von 2-(S)-Benzyl-4-oxo-4-(cis-perhydroisoindol-2-yl)-buttersäure durch enantioselektive katalytische Hydrierung von Isoindolin, gefolgt von einer Reaktion der erhaltenen Verbindung mit dem Anhydrid der Formel (II): 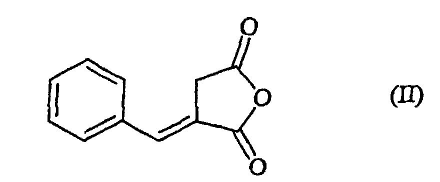 gefolgt von einer katalytischen Hydrierung der in dieser Weise erhaltenen Verbindung (III) in Gegenwart eines asymmetrischen Katalysators: 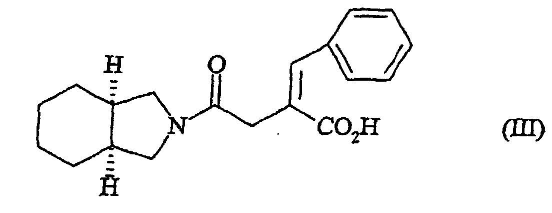 **dadurch gekennzeichnet, daß** das als Ausgangsmaterial eingesetzte Isoindolin nach dem Verfahren von Anspruch 1 hergestellt wird.
